Europäisches Patentamt

European Patent Office

Office européen des brevets

(19)

(11) Veröffentlichungsnummer: **0 143 209 B1**

(12) **EUROPÄISCHE PATENTSCHRIFT**

(45) Veröffentlichungstag der Patentschrift: **27.11.91**

(51) Int. Cl.⁵: **G01N 33/563**, G01N 33/68

(21) Anmeldenummer: **84110406.0**

(22) Anmeldetag: **31.08.84**

(54) **Verfahren zur immunologischen Bestimmung von extrazellulären Matrixproteinen in Körperflüssigkeiten, unter Verwendung monovalenter Antikörperfragmente.**

(30) Priorität: **01.09.83 DE 3331627**

(43) Veröffentlichungstag der Anmeldung:
**05.06.85 Patentblatt 85/23**

(45) Bekanntmachung des Hinweises auf die
Patenterteilung:
**27.11.91 Patentblatt 91/48**

(84) Benannte Vertragsstaaten:
**AT BE CH DE FR GB IT LI LU NL SE**

(56) Entgegenhaltungen:
**EP-A- 0 021 152
DE-A- 2 643 207
DE-A- 2 743 444
US-A- 4 235 869**

(73) Patentinhaber: **Max-Planck-Gesellschaft zur Förderung der Wissenschaften e.V.
Bunsenstrasse 10
W-3400 Göttingen(DE)**

(72) Erfinder: **Timl, Rupert, Dr.
Julius Haerlin-Str. 3
W-8035 Gauting(DE)**

(74) Vertreter: **Weickmann, Heinrich, Dipl.-Ing. et al
Patentanwälte Dipl.-Ing. H.Weickmann
Dipl.-Phys.Dr. K.Fincke Dipl.-Ing.
F.A.Weickmann Dipl.-Chem. B. Huber Dr.-Ing.
H. Liska Dipl.-Phys.Dr. J. Prechtel Postfach
860820
W-8000 München 86(DE)**

**Beschreibung**

Die Erfindung betrifft ein Verfahren zur immunologischen Bestimmung von extrazellulären Matrix-Proteinen in Körperflüssigkeiten, die eine nichtparallele Inhibitionskurve zu einem Referenzinhibitor zeigen, unter Verwendung monovalenter Antikörperfragmente sowie hierfür geeignete monovalente Antikörperfragmente.

Immunologische Verfahren sind Standardverfahren zum spezifischen und quantitativen Nachweis von Proteinen und Peptiden in Körperflüssigkeiten, wie z. B. Serum oder Urin und in Gewebeextrakten, welche sich durch eine hohe Empfindlichkeit und Spezifität auszeichnen und daher eine große Bedeutung für die klinische Diagnostik erlangt haben.

Der Radio-Immuno- Assay beruht darauf, daß man mit Hilfe möglichst hochgereinigter Antigene (z. B. Proteine oder Peptide) in geeigneten Versuchstieren ein Antiserum herstellt. Das Antiserum wird zusammen mit einer vorgegebenen Menge radioaktiv markierten Antigens und der zu untersuchenden Körperflüssigkeit inkubiert. Enthält die Körperflüssigkeit das entsprechende Antigen, so findet man im gebildeten Immunkomplex weniger Radioaktivität, da ein Teil des radioaktiv markierten Antigens durch das in der Körperflüssigkeit vorhandene, nichtmarkierte Antigen verdrängt wird. Diese Verdrängung (Inhibition) des radioaktiv markierten Antigens ist proportional zu der zugegebenen Menge Körperflüssigkeit. Die Verdrängungsreaktion wird in einem separaten Ansatz durch Zugabe von gereinigtem Antigen bekannter Konzentration zu dem System markiertes Protein/Antise rum kalibriert. Üblicherweise beobachtet man für beide Inhibitoren (gereinigtes Protein bzw. Körperflüssigkeit) parallele Inhibitionskurven, so daß sich die Menge Antigen in der Körperflüssigkeit aus dem Vergleich beider Kurven errechnen läßt.

Bei dem auf demselben Prinzip beruhenden Enzym-Immuno-Assay, verwendet man als Partner der Immunreaktion Enzyme (EIA, ELISA). Weiterhin besteht die Möglichkeit,die Markierung mit Fluorochromen vorzunehmen.

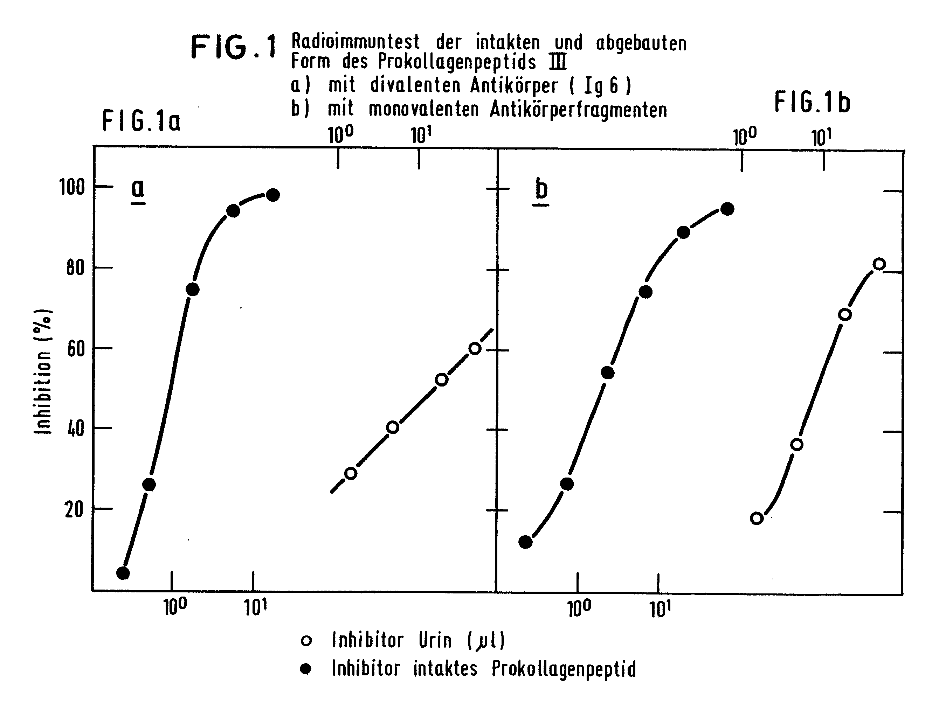

Bei extracellulären Matrixproteinen wurde jedoch beobachtet, daß die Inhibitionskurven von gereinigtem Referenzprotein und Körperflüssigkeit einen nichtparallelen Verlauf zeigen (Fig. 1a). Dabei ist üblicherweise die Kurve der Körperflüssigkeit weniger steil als die des Referenzproteins. Das bedingt naturgemäß eine beträchtliche Unsicherheit in der quantitativen Auswertung oder macht diese sogar unmöglich.

Eine mögliche Erklärung für dieses Phänomen ist die Annahme, daß die Körperflüssigkeit nicht das intakte Protein, sondern eine modifizierte (z. B. proteolytisch abgebaute) Form enthält, die eine geringere Affinität für den Antikörper aufweist. Andere Erklärungsmöglichkeiten beinhalten eine Reihe nicht näher charakterisierte technische Parameter, wie z. B. hohe Proteinkonzentration oder Viskosität der zu testenden Körperflüssigkeit, die in unspezifischer Weise zu einer Veränderung des Inhibitionsprofils beitragen.

Eine typische Beobachtung zu diesem Problem wurde bei der Entwicklung eines Radio-Immuno-Assays für Aminopropeptid (Prokollagenpeptid Typ III) gemacht (Rohde et al., Eur. J. Clin. Invest. 9, 451-459 (1979)). Hier konnte das intakte Peptid im Serum nachgewiesen werden und zeigte im Vergleich zu authentischem Aminopropeptid eine parallele Inhibitionskurve. Urin hingegen enthält eine abgebaute Form des Peptids und seine immunologische Aktivität ist durch eine nichtparallele Inhibitionskurve charakterisiert (Fig. 1a). Eine quantitative Bestimmung des Prokollagens in Urin ist damit nicht möglich.

Der Erfindung liegt daher die Aufgabe zugrunde, dieses Problem zu lösen und ein Verfahren zu schaffen, das eine einfache quantitative Bestimmung von extrazellulären Matrixproteinen, die unter ungünstigen technischen Bedingungen vorliegen, ermöglicht.

Gelöst wird diese Aufgabe erfindungsgemäß durch ein Verfahren zur immunologischen Bestimmung von extrazellulären Matrixproteinen in Körperflüssigkeiten, welches dadurch gekennzeichnet ist, daß als Antiserum spezifische monovalente Antikörperfragmente verwendet werden.

Die Verwendung von monovalenten Antikörperfragmenten bei immunologischen Untersuchungen ist aus der Literatur bekannt. Jedoch wurde bisher angenommen, daß normalerweise kein Affinitätsunterschied zwischen bivalenten Antikörpern und monovalenten Antikörperfragmenten besteht. So fand man z. B. keinen Unterschied in der Hemmwirkung von Antikörpern gegen Creatinkinase auf die Creatinkinaseaktivität, wenn man dazu bivalente oder monovalente Antikörper verwendete (U. Würzburg, Kontakte 3, S. 10-17 (1978)).

Es war daher überraschend, daß die Verwendung monovalenter Antikörperfragmente anstelle von bivalenten Antikörpern zu einer deutlichen Verbesserung der Inhibitionskurve bei immunologischen Bestimmungen von extrazellulären Matrixproteinen führt bzw. in einigen Fällen eine immunologische Bestimmung überhaupt erst ermöglicht.

Am Beispiel des Aminopropeptids zeigt eine vergleichende Analyse von Antikörper und monovalentem Antikörperfragment im Immun-Test mit intaktem Prokollagenpeptid als markiertem Protein und als Referenzinhibitor in beiden Fällen eine vergleichbare Nachweisempfindlichkeit. Urinproben zeigen im Test mit

2

EP 0 143 209 B1

dem monovalenten Antikörperfragment eine parallele Inhibitionskurve und erlauben damit eine verläßliche quantitative Bestimmung des Prokollagenpeptids Typ III in diesem Material (Fig. 1b).

Aber auch bei der Bestimmung von Aminopropeptid in Seren ist die Verwendung von monovalenten Antikörperfragmenten anstelle von bivalenten Antikörpern vorteilhaft. Die Analyse der Seren zeigt eine fünf- bis sechsfach höhere Konzentration an Prokollagenpeptid bei Messung mit dem Antikörperfragment-Test im Vergleich zur Messung der gleichen Seren mittels bivalenter Antikörper. Die biochemische Analyse der Seren durch Molekularsiebchromatographie zeigt, daß etwa ein Fünftel des Aminopropeptids als intaktes Material (Molekulargewicht 45000), der Rest als degradierte Form (Molekulargewicht 10000) vorliegt. Der Test mit bivalentem Antikörper weist hauptsächlich nur intaktes Aminopropeptid nach, während durch den Antikörperfragment-Test beide Formen erfaßt werden, wodurch die Empfindlichkeit der Methode verbessert wird. Die umfassende Analyse des Aminopropeptids in der inaktiven und in der degradierten Form mit Hilfe des Antikörperfragment-Tests stellt eine wertvolle Erweiterung für Serum- und Urinanalysen, z. B. zur Diagnose von Leberfibrose, dar.

Ein Beispiel für ein weiteres Protein der angegebenen Klasse, dessen Bestimmung in Körperflüssigkeiten für die Diagnose von Diabetes und Lebererkrankungen von Bedeutung ist, ist das Humanlaminin P1. Auch hier konnte durch die Verwendung von monovalenten Antiköperfragmenten eine Verbesserung der immunologischen Bestimmungsmethode erzielt werden. Andere Beispiele von extracellulären Matrixproteinen, für welche sich die Erfindung eignet, sind Kollagene, Prokollagene und Fibronectin.

Zur immunologischen Bestimmung von extracellulären Matrixproteinen mit Hilfe von monovalenten Antikörperfragmenten gemäß Erfindung können alle bekannten immunologischen Methoden angewandt werden. Beispielsweise können alle Ausführungsformen des RIA-Testes, z. B. sequentielle Sättigungsanalyse oder Gleichgewichtsanalyse, Markierung mit Chloramin T oder Bolton-Hunter-Reagenz, (Lit: Felber, Meth. Biochem. Anal. 22, 1, 1974; Skelley et al., Clin. Chem. 19, 146-186, 1973) eingesetzt werden. Das betrifft auch den Enzym-Immuntest. Eine Einschränkung betrifft die Separierungstechnik soweit die Tests auf dem Fc-Anteil des Antikörpers beruhen (fehlt im Fab), wie z. B. die Komplexierung von Antikörpern mit Protein A von Staphylokokken. Andere Separierungsverfahren, z. B. zweiter Antikörper in gebundener oder löslicher Form sind äquivalent. Ebenso sind Verfahren mit fluorochrommarkierten Antikörpern geeignet.

Die Herstellung der Antiseren kann in üblicher Weise durch Injektion des Antigens in Versuchstiere, vorzugsweise Kaninchen, erfolgen. Zweckmäßig wird dabei in Gegenwart von komplettem Freund'schem Adjuvans gearbeitet. Es können die in derartigen Fällen üblichen Antigenmengen verabreicht werden. Als besonders geeignete Dosis bei Verwendung von Kaninchen erwiesen sich 0,5 bis 1 mg pro Tier. Das gebildete Antiserum wird dann in der dem Fachmann bekannten Weise gewonnen und kann weiter gereinigt werden, beispielsweise nach Methoden der Affinitätschromatographie.

Die Präzipitation mit einem zweiten Antikörper gegen Immunglobulin ist eine besonders gut geeignete Ausführungsform für den Fab-Test. Die Antiseren enthalten für diesen Zweck genügend Antikörper gegen Fab. Eine Immunisierung mit Fab ist deshalb nicht notwendig. Besonders günstige Ergebnisse wurden mit Ziegenantiseren erzielt.

Die monovalenten Antikörperfragmente können aus den gereinigten Antikörpern oder aus der Immunglobulinfraktion der Antiseren nach den üblichen Verfahren hergestellt werden. In einer bevorzugten Ausführungsform wird der Antikörper durch Pepsin abgebaut. Anschließend wird reduziert und man erhält auf diese Weise das Fab'-Fragment. In einer weiteren bevorzugten Ausführungsform der Erfindung werden Fab-Fragmente verwendet, die durch Papainabbau unter reduzierenden Bedingungen hergestellt werden.

Durch die Erfindung wird erreicht, daß sonst schwer bestimmbare extracelluläre Matrixproteine, mit hoher Genauigkeit und sehr großer Empfindlichkeit nach immunologischen Methoden bestimmt werden können, wobei die Markierung radioaktiv erfolgen kann oder durch eine der anderen bekannten Markierungen ersetzt werden kann. Für bestimmte Proteine der angegebenen Klasse ermöglicht die Erfindung erstmals eine immunologische Bestimmung.

Die folgenden Beispiele erläutern die Erfindung näher:

**Beispiel 1**

Das Prokollagen-III-Peptid wird aus foetaler Kalbshaut gewonnen und gereinigt. Antiseren wurden im Kaninchen gegen Prokollagen III oder Prokollagen-III-Peptid hergestellt, wie in der EP-A-4940 beschrieben.

Für den RIA-Test werden 25 μg des Prokollagen-III-Peptides mit 0,5-1mCi Jod-125 unter Verwendung der Chloramin-T-Methode markiert und nicht gebundenes Jod durch Dialyse entfernt. Aus den Antiseren wird die Immunglobulin-G-Fraktion an DEAE-Zellulose oder der spezifische Antikörper durch Affinitätschromatographie an einer Prokollagen-III-Säule nach Standardverfahren gewonnen. Diese Materialien wurden durch Pepsinabbau und Reduktion in monovalente Fab' Fragmente (Nisonoff et al., Arch. Biochem.Biophys.

3

89, 230-244 (1960)) oder durch Papainabbau in monovalente Fab Fragmente (Mage, Meth. Enzymol. 70, 142-150, 1980) überführt. Die Fragmente wurden anschließend durch Molekularsiebchromatographie an Sephacryl S-200$^R$ (Säule 1,5x120cm in 0,2M Ammoniumbicarbonat) von nicht-degradiertem Antikörper oder bivalenten Fragmenten abgetrennt.

Die Durchführung des RIA-Testes erfolgt dann in Gegenwart eines Detergens (0,04% Tween®20) wie in EU-PS 4940 beschrieben. Die Fällung der Immunkomplexe wird dann mit einem zweiten Antikörper gegen Immunglobulin G vorgenommen, sie kann auch nach einer anderen dem Fachmann geläufigen Methode durchgeführt werden.

**Beispiel 2**

Es wird wie im Beispiel 1 beschrieben vorgegangen, jedoch ausgehend von Lamininfragment P1, welches wie in EP-A-4940 beschrieben, gewonnen worden war. Markierung, Reinigung und RIA-Test werden wie im Beispiel 1 beschrieben, durchgeführt.

**Beispiel 3**

Die Mengen von Aminopropeptid in biologischen Proben wurden erfindungsgemäß mit Fab' Fragmenten unter Verwendung des Peptids Col 1-3 als Referenzinhibitor gemessen. Die Analyse des Serums von 53 normalen erwachsenen Personen im Alter von 20 bis 65 Jahren sowie 7 Urinproben zeigten gute Reproduzierbarkeit, gewöhnlich mit 10 bis 20% Variation. Tabelle 1 zeigt die Ergebnisse.

T a b e l l e 1

Reproduzierbarkeit der mit Fab-RIA erhaltenen Werte für zwei zufällig ausgewählte Serum- und Urin-Proben.

| Probe (Test A oder B) a) | | Procollagenpeptid-Menge (ng/ml) b) gemessen bei Verdünnung 1:5 1:10 1:20 | | | Mittel + S.D. |
|---|---|---|---|---|---|
| Serum Nr. 23 | A | 26 | 30 | 39 | 32 ± 7 |
|  | B | 30 | 31 | 28 | 30 ± 2 |
| Serum Nr. 24 | A | 30 | 28 | 29 | 29 ± 1 |
|  | B | 27 | 21 | 29 | 26 ± 4 |
| Urin Nr. 1 | A | 59 | 88 | 109 | 85 ± 25 |
|  | B | 93 | 89 | 99 | 94 ± 5 |
| Urin Nr. 14 | A | 47 | 54 | 56 | 52 ± 5 |
|  | B | 62 | 55 | 52 | 56 ± 5 |

a) Sequentielle Sättigungstests, ausgeführt bei verschiedenen Gelegenheiten mit zwei verschiedenen Präparaten von markiertem Antigen, jedoch gleichem Fab-Fragment und Bezugsinhibitor.

b) Mittel aus je 2 Bestimmungen pro Verdünnung

**Patentansprüche**

1. Verfahren zur immunologischen Bestimmung von extracellulären Matrix-Proteinen in Körperflüssigkeiten, **dadurch gekennzeichnet,** daß als Antiserum spezifische monovalente Antikörperfragmente verwendet werden.

2. Verfahren nach Anspruch 1, **dadurch gekennzeichnet,** daß eine radioimmunologische Bestimmung durchgeführt wird.

3. Verfahren nach einem der Ansprüche 1 bis 2, **dadurch gekennzeichnet,** daß man monovalente Antikörperfragmente verwendet, die durch Pepsinabbau des Immunglobulins und nachfolgende Reduktion hergestellt wurden.

4. Verfahren nach einem der Ansprüche 1 bis 2, **dadurch gekennzeichnet,** daß man monovalente Antikörperfragmente verwendet, die durch Papainabbau unter reduzierenden Bedingungen hergestellt wurden.

5. Verfahren nach einem der Ansprüche 1 bis 4, **dadurch gekennzeichnet,** daß Prokollagen-III-peptid bestimmt wird.

6. Verfahren nach einem der Ansprüche 1 bis 4, **dadurch gekennzeichnet,** daß Laminin P1 bestimmt wird.

7. Verfahren nach einem der vorhergehenden Ansprüche, **dadurch gekennzeichnet,** daß Antigenfragmente im Urin bestimmt werden.

8. Verfahren nach einem der vorhergehenden Ansprüche, **dadurch gekennzeichnet,** daß Antigenfragmente im Serum bestimmt werden.

## Claims

1. Process for the immunological determination of extracellular matrix proteins in body fluids, characterised in that specific monovalent antibody fragments are used as antiserum.

2. Process according to claim 1, characterised in that a radioimmunological determination is carried out.

3. Process according to one of claims 1 to 2, characterised in that one uses monovalent antibody fragments which have been prepared by pepsin breakdown of the immune globulin and subsequent reduction.

4. Process according to one of claims 1 to 2, characterised in that one uses monovalent antibody fragments which have been prepared by papain breakdown under reducing conditions.

5. Process according to one of claims 1 to 4, characterised in that procollagen III peptide is determined.

6. Process according to one of claims 1 to 4, characterised in that laminine P1 is determined.

7. Process according to one of the preceding claims, characterised in that antigen fragments are determined in urine.

8. Process according to one of the preceding claims, characterised in that antigen fragments are determined in serum.

## Revendications

1. Procédé de détermination immunologique de protéines de matrice extracellulaires dans des liquides corporels, caractérisé en ce que des fragments d'anticorps monovalents spécifiques sont utilisés en tant qu'antisérum.

2. Procédé selon la revendication 1, caractérisé en ce qu'on effectue un dosage radio-immunologique.

3. Procédé selon l'une des revendications 1 à 2, caractérisé en ce qu'on emploie des fragments

6

d'anticorps monovalents qui ont été préparés par scission avec de la pepsine de l'immonuglobuline et réduction subséquente.

4. Procédé selon l'une des revendications 1 à 2, caractérisé en ce qu'on emploie des fragments d'anticorps monovalents qui ont été préparés par scission avec de la papaïne dans des conditions réductrices.

5. Procédé selon l'une des revendications 1 à 4, caractérisé en ce que l'on dose le peptide de procollagène III.

6. Procédé selon l'une des revendications 1 à 4, caractérisé en ce que l'on dose la laminine P1.

7. Procédé selon l'une des revendications précédentes, caractérisé en ce que l'on dose des fragments d'antigène dans l'urine.

8. Procédé selon l'une des revendications précédentes, caractérisé en ce que l'on dose des fragments d'antigène dans le sérum.

FIG.1 Radioimmuntest der intakten und abgebauten
Form des Prokollagenpeptids III
a) mit divalenten Antikörper ( Ig 6 )
b) mit monovalenten Antikörperfragmenten

FIG.1a

FIG.1b

o Inhibitor Urin (µl)
● Inhibitor intaktes Prokollagenpeptid

EP 0 143 209 B1